# EUROPEAN PATENT APPLICATION

(11) **EP 3 834 805 A1**
(43) Date of publication of application: **16.06.2021**
(21) Application number: 20150932.0
(22) Date of filing: 09.01.2020
(51) Int. Cl.: A61K 6/61, A61K 6/71, A61K 6/889

(54) **DENTAL RESIN MODIFIED GLASS-IONOMER COMPOSITION AND KIT COMPRISING SAID COMPOSITION**

(30) Priority: 12.12.2019 US 201962947152 P
(71) Applicant: DENTSPLY SIRONA Inc., York, 17401-2991 (US); DENTSPLY DETREY GmbH, 78467 Konstanz (DE)
(72) Inventor: Szillat, Florian, 78462 Konstanz (DE); Neuhaus, Kira, 40549 Düsseldorf (DE); Pohle, Sven, 78467 Konstanz (DE); Lui, Huaibing, Dover, DE 19904 (US)
(74) Representative: Dietz, Mirko

(57) **Abstract**

The present invention is related to a dental resin modified glass-ionomer composition comprising
(a) water;
(b) at least one polycarboxylic acid;
(c) at least one compound having at least one phosphorous atom; and
(d) at least one bisacrylamide having the following formula (I):

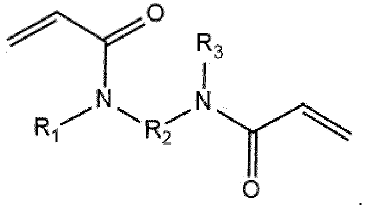

The present invention is further related to a kit comprising such a dental resin modified glass-ionomer composition.

## Description

### Field of the Invention

The present invention relates to a dental resin modified glass-ionomer composition.

The present invention is further directed to a kit comprising such a dental resin modified glass-ionomer composition.

### Background of the Invention

Dental cements are used to attach prosthetic devices such as inlays, onlays, crowns, or posts to tooth structures so that a diseased tooth can restore its function and aesthetics. Dental cements can also be used to attach orthodontic devices, such as orthodontic brackets or an orthodontic band, to a tooth structure to correct misalignment of teeth or adjust spaces between teeth. Dental cements can also be used as root canal sealants.

When cementing prosthetic and orthodontic devices to tooth structures, there are a variety of classes of dental cements available to dental practitioners to choose from and they include, for example, (1) a zinc phosphate cement, (2) a zinc carboxylate cement, (3) a zinc oxide eugenol (ZOE) cement, (4) a glass-ionomer cement, (5) a self-adhesive and adhesive resin cements, and (6) a resin modified glass-ionomer (RMGI) cement. Each type of cement has its own concomitant advantages and shortcomings, as discussed next.

For example, the zinc phosphate cement (1) utilizes the acid-base setting reaction between zinc oxide and phosphoric acid. Generally, the zinc phosphate cement is provided as a powder/liquid system, which requires manual mixing of the powder and liquid components prior to application. Manual mixing can be a messy process and often lacks consistency due to improper weighing/rationing of powder and liquid components. In addition, zinc phosphate cements have poor adhesive strength to tooth structure, high optical opacity (poor aesthetics), and have a high solubility in the oral environment. Zinc phosphate cements also possess a substantially low initial pH, which can cause irritation or sensitivity during early stages of setting.

The zinc carboxylate cement (2) utilizes the acid-base setting reaction between zinc oxide and polycarboxylic acid. Comparatively, zinc carboxylate cements are gentler to pulp tissue and have improved adhesive property toward tooth structure, as compared to their zinc phosphate counterparts. However, zinc carboxylate cements have a short working time, high optical opacity, low mechanical strength, and require an additional conditioning step prior to cementation.

The zinc oxide eugenol (ZOE) cement (3) utilizes the acid-base setting reaction between zinc oxide and eugenol in the presence of water to form a zinc eugenolate chelate. A ZOE cement has low mechanical strength, poor adhesive properties, poor aesthetics, and high solubility in water, thereby making it suitable for use only as a provisional or temporary cement.

The self-adhesive and adhesive resin cements (5) utilize free-radical polymerization setting reaction of (meth)acrylate monomers. Self-adhesive and adhesive resin cements are generally reinforced with inorganic glass fillers and possess very good mechanical strength, favorable aesthetic properties, and low solubility in water. Self-adhesive resin cements have good bond strengths to tooth structure without using any bonding agent and adhesive resin cements demonstrate excellent bond strengths to tooth structure, when used in combination with a bonding agent. As a result, self-adhesive and adhesive resin cements are best suited for cementing aesthetic, yet brittle ceramic restorations. Nevertheless, self-adhesive and adhesive resin cements are rather hydrophobic and sensitive to water/saliva contamination. Specially, adhesive resin cements are also quite technique sensitive and have the potential to cause post-operative sensitivity due to their complicated bonding protocol requiring a separate bonding agent.

The glass-ionomer cement (6) utilizes the acid-base setting reaction between a polycarboxylic acid and a fluoroaluminosilicate filler. In comparison to the zinc phosphate cements, the glass-ionomer cements generally demonstrate improved adhesive property, reduced solubility in water, improved mechanical strength, and have the added benefit of cariogenic properties due to long-term sustained release of fluoride. However, glass-ionomer cements have poor aesthetics as well as desiccation during the early stage of setting. When compared to resin cements, the glass-ionomer cements are quite brittle, and their bond strength to tooth structure is substantially lower.

Various efforts have been made to combine resin cement chemistry with glass-ionomer cement chemistry to form resin modified glass-ionomer (RMGI) cements (7). Akahane et al. (U.S. Pat. No. 5,063,257) incorporated polymerizable monomers, a initiator, and a surfactant into a glass-ionomer composition to make the resulting RMGI composition curable through both an acid-base reaction and a free-radical polymerization. Mitra et al. (U.S. Pat. Nos. 5,130,347 and 5,925,715) incorporated a photoinitiator system into a glass-ionomer composition and added polymerizable groups to the polycarboxylic acid through an amide linkage, to make the resulting RMGI composition curable through both an acid-base reaction and a free radical photo-polymerization. Jandourek (EP 0,329,268A2) incorporated a photoinitiator system, polymerizable monomers, and a polymerizable polycarboxylic acid into a glass-ionomer composition to make the resulting RMGI composition curable through both an acid-base reaction and a free radical photo-polymerization. Mitra et al. (U.S. Pat. No. 5,154,762) incorporated a redox initiator system, along with polymerizable monomers and polymerizable polycarboxylic acid, into a glass-ionomer composition to make the resulting RMGI composition curable through an acid-base reaction and a free radical polymerization, either by a photoinitiator or a redox initiator system. Nakaseko (U.S. Pat. No. 6,214,101) made a paste/paste RMGI composition by incorporating polymerizable monomers and encapsulated polymerization initiators into a glass-ionomer composition.

Accordingly, RMGI cement compositions combine the setting chemistries from both glass-ionomer cements and resin cements. RMGI cement compositions retain the benefit of sustained long-term fluoride release, and provide improved mechanical strength, fracture toughness, and adhesive properties over those of traditional glass-ionomer cements alone. And due to the hydrophilic and self-adhesive nature, RMGI cement compositions are generally less sensitive to moisture/saliva contamination and are less technique sensitive, as compared to self-adhesive and adhesive resin cements.

However, the bond strengths to tooth structure of prior RMGI cement compositions are still significantly lower than a self-adhesive and adhesive resin cements.

### Objective of the present Invention

In view of the prior art, it was thus an object of the present invention to provide a dental resin modified glass-ionomer (RMGI) composition, which shall not exhibit the aforementioned shortcomings of the known prior art RMGI compositions.

What is needed therefore is a way to further improve the adhesive properties of RMGI compositions.

Furthermore, it was an object to provide a RMGI composition that shows significant increased bong strength compared to other conventional RMGI compositions.

### Summary of the Invention

These objects and further objects which are not stated explicitly but are immediately derivable or discernible from the connections discussed herein by way of introduction are achieved by a dental resin modified glass-ionomer composition having all features of claim 1. Appropriate modifications to the inventive dental resin modified glass-ionomer composition are protected in dependent claims 2 to 12. Further, claim 13 comprises a kit comprising such a dental resin modified glass-ionomer composition while dependent claims 14 and 15 are representing preferred embodiments of said kit.

The present invention accordingly provides a dental resin modified glass-ionomer composition characterized in that the dental resin modified glass-ionomer composition comprises
(a) water;
(b) at least one polycarboxylic acid;
(c) at least one compound having at least one phosphorous atom selected from the group consisting of a (meth)acrylate having at least one phosphate group, a (meth)acrylate having at least one phosphonate group, a (meth)acrylamide having at least one phosphate group, a (meth)acrylamide having at least one phosphonate group, an acrylic ether having at least one phosphate group, and an acrylic ether having at least one phosphonate group; and
(d) at least one bisacrylamide having the following formula (I): 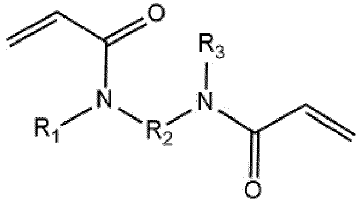 wherein
   R₁ = hydrogen, a C₁ to C₁₈ alkyl group, a C₃ to C₁₈ alkylene group comprising at least one carbon-carbon double bond, a C₃ to C₁₈ cycloalkyl group, or a C₅ to C₁₈ aryl group;
   R₂ = a difunctional C₁ to C₁₈ alkyl group, a difunctional C₄ to C₁₈ alkylene group comprising at least one carbon-carbon double bond, a difunctional C₃ to C₁₈ cycloalkyl group, a difunctional C₅ to C₁₈ aryl group, or a difunctional C₁ to C₁₈ hydrocarbon moiety being substituted with at least one, preferably one or two, acrylamide or acrylate group(s); and
   R₃ = hydrogen, a C₁ to C₁₈ alkyl group, a C₃ to C₁₈ alkylene group comprising at least one carbon-carbon double bond, a C₃ to C₁₈ cycloalkyl group, or a C₅ to C₁₈ aryl group.

It is thus possible in an unforeseeable manner to provide a dental resin modified glass-ionomer (RMGI) composition, which does not exhibit the aforementioned shortcomings of the known prior art RMGI compositions.

Furthermore, the RMGI compositions of the present invention show significant increased bong strength compared to other conventional RMGI compositions.

In addition, the present invention further improves the adhesive properties of known RMGI compositions.

The at least one compound having at least one phosphorous atom provides a strong acidity and has thereby a high effect for dissolving a smear layer of a tooth surface and for tooth decalcification. Particularly, a (meth)acrylate having at least one phosphate group can exercise an effect for improving adhesive property to enamel.

### Detailed Description of the Invention

The expression "substantially free" means in the context of the present invention a concentration of less than 5 weight percent, preferably less than 2.5 weight percent, and more preferably less than 1 weight percent based on the total weight of the dental (RMGI) composition.

As used herein, the term "resin modified glass-ionomer (RMGI)" and the alternative term "resin modified glass-ionomer (RMGI) cement" are exchangeable in the context of the present invention.

The term "(meth)acrylate" in the context of the present disclosure is meant to refer to the acrylate as well as to the corresponding methacrylate.

The term "hydrocarbon moiety" refers to a linear or branched saturated or unsaturated hydrocarbon chain of the respective length. Such a hydrocarbon moiety may be substituted further with one or more substituents such as alkylene, alkoxy, acrylamide, acrylate, nitrile, aryl, cycloalkyl, and hydroxyl.

The term "alkyl group" refers to a linear or branched saturated hydrocarbon chain of the respective length. This term can be exemplified by groups such as methyl, ethyl, n-propyl, iso-propyl, n-butyl, iso-butyl, sec-butyl, t-butyl, n-pentyl, isopentyl, neopentyl, n-hexyl, n-decyl, dodecyl, tetradecyl, and the like. Such alkyl groups may be substituted further with one or more substituents such as alkylene, alkoxy, nitrile, aryl, cycloalkyl, and hydroxyl.

The term "alkylene group" refers to a linear or branched hydrocarbon chain of the respective length, which comprises at least one carbon-carbon double bond. Such alkylene groups may be substituted further with one or more substituents such as alkyl, alkoxy, nitrile, aryl, cycloalkyl, and hydroxyl.

The term "aryl group" refers to an aromatic, heterocyclic, fused aromatic, fused heterocyclic, biaromatic, or bihetereocyclic ring systems having the given specific number of carbon atoms. Broadly defined, "aryl", as used herein, includes 5 to 18-membered single-ring aromatic groups that may include from zero to four heteroatoms, for example, benzene, pyrrole, furan, thiophene, imidazole, oxazole, thiazole, triazole, pyrazole, pyridine, pyrazine, pyridazine, pyrimidine, and the like. Those "aryl" groups having heteroatoms in the ring structure may also be referred to as "heteroaryl" or "heterocycles" or "heteroaromatics". The aromatic ring can be substituted at one or more ring positions with one or more substituents such as halogen, alkyl, alkylene, cycloalkyl, hydroxyl, and alkoxy.

The term "cycloalkyl group" refers to monocyclic or polycyclic cycloalkyl group. Examples include cyclopropyl, cyclobutyl, cyclopentyl, cyclohexyl, cycloheptyl, and cyclooctyl. Examples of polycyclic cycloalkyl groups include, for example admantyl, norbornyl, decalinyl, 7,7-dimethyl-bicyclo[2.2.1]heptanyl, tricyclo[5.2.1.02,6]decyl and the like. Such a cycloalkyl group may be substituted further with one or more substituents such as alkyl, alkoxy, nitrile, aryl, cycloalkyl, and hydroxyl.

For component (a), water provides a medium needed for the ionic acid-base reaction to take place between the polycarboxylic acid (b) and the fluoroaluminosilicate filler (f). According to embodiments of the present invention, water (a) may be present in an amount from 0.5 to 40 weight percent, preferably from 1.0 to 30 weight percent, and more preferably from 2.0 to 25 weight percent based on the total weight of the dental RMGI composition.

For component (c), at least one compound having at least one phosphorous atom, which is selected from the group consisting of a (meth)acrylate having at least one phosphate group, a (meth)acrylate having at least one phosphonate group, a (meth)acrylamide having at least one phosphate group, a (meth)acrylamide having at least one phosphonate group, an acrylic ether having at least one phosphate group, and an acrylic ether having at least one phosphonate group; is comprised by the inventive dental RMGI composition.

Such a phosphorous containing polymerizable monomer is commonly only comprised in water-free self-adhesive resin cements (also called SARC's). These SARC's provide good aesthetics, good (in comparison to a RMGI composition superior) adhesive abilities to dentin but suffers from bad applicability for the customer.

Such a phosphorous containing polymerizable monomer is up to now never comprised in water containing RMGI compositions, which provide bad aesthetics, bad (in comparison to a resin cement inferior) adhesive abilities to dentin, but good applicability for the customer.

Such a compound having at least one phosphorous atom can be a (meth)acrylate having at least one phosphate group. The term "phosphate group" includes in the present invention also the possibility of having a phosphoric acid group, wherein each R residue of each oxygen atom of the phosphate group is hydrogen, such as shown below by MEP, GDMP, MDP, or PENTA-P.

Certain specific examples are given herewith in the following:

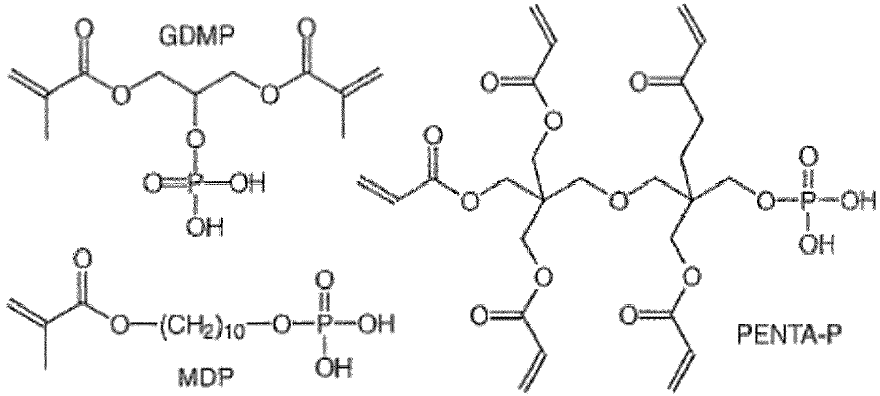

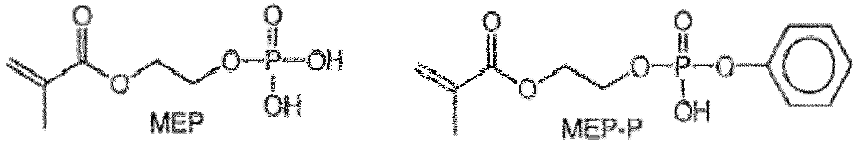

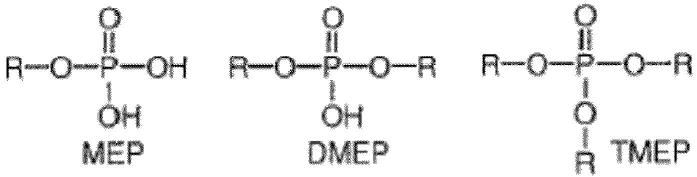

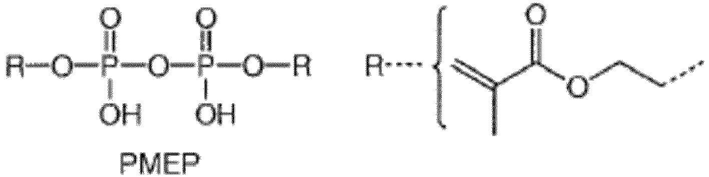

Such a compound having at least one phosphorous atom can be also a (meth)acrylamide having at least one phosphate group, wherein (compared to the (meth)acrylate) the oxygen atom of each (meth)acrylic ester group is substituted by a nitrogen atom forming thereby an (meth)acrylamide instead of an (meth)acrylate.

Such a compound having at least one phosphorous atom can be also a (meth)acrylate having at least one phosphonate group. The term "phosphonate group" includes in the present invention also the possibility of having a phosphonic acid group, wherein each R residue of each oxygen atom of the phosphonate group is hydrogen, such as shown below by MAPA-1 or MAPA-2.

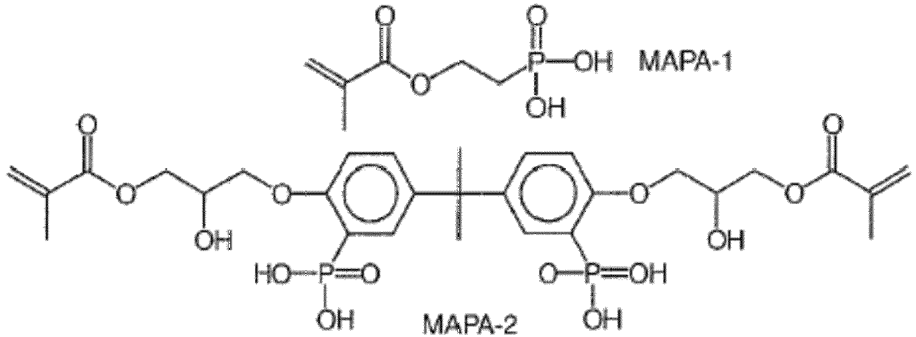

Such a compound having at least one phosphorous atom can be also a (meth)acrylamide having at least one phosphonate group, wherein (compared to the (meth)acrylate) the oxygen atom of each (meth)acrylic ester group is substituted by a nitrogen atom forming thereby an (meth)acrylamide instead of an (meth)acrylate.

Such a compound having at least one phosphorous atom can be also an acrylic ether having at least one phosphonate group. Some examples are given below:

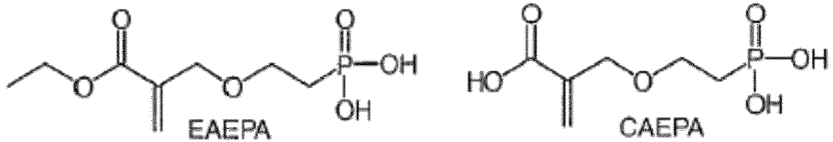

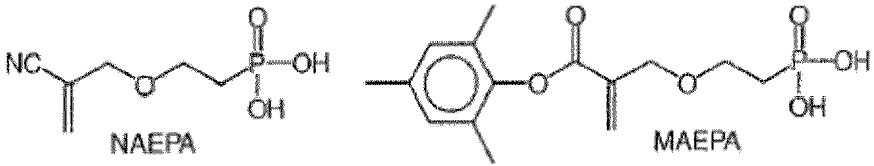

Such a compound having at least one phosphorous atom can be also an acrylic ether having at least one phosphate group.

All compounds having at least one phosphorous atom of component (c) being comprised in the dental RMGI composition (or in all parts of a kit, if a kit is present) are provided in an amount of 2 to 30 weight percent, preferably 3 to 20 weight percent, and more preferably 4 to 10 weight percent based on the total weight of the dental RMGI composition.

In one embodiment, the at least one compound having at least one phosphorous atom is selected from the group consisting of 2-(meth)acryloyloxyethyl-dihydrogen phosphate, bis[2-(meth)acryloyloxyethyl]hydrogen phosphate, 2-(meth)acryloyloxyethylphenylhydrogen phosphate, 6-(meth)acryloyloxyhexyldihydrogen phosphate, 6-(meth)acryloyloxyhexylphenylhydrogen phosphate, 10-(meth)acryloyloxydecyldihydrogen phosphate, 1,3-di(meth)acryloylpropane-2-dihydrogen phosphate, 1,3-di(meth) acryloylpropane-2-phenylhydrogen phosphate, dipentaerythrolpentaacryloyl dihydrogen phosphate, ethyl 2-[5-dihydrogen phosphoryl-5.2-dioxa pentyl]acrylate, and bis[5-{2-(meth)acryloyloxyethoxycarbonyl} heptyl]hydrogen phosphate.

In a preferred embodiment thereof, the at least one compound having at least one phosphorous atom is selected from the group consisting of 10-methacryloyloxydecyldihydrogen phosphate (MDP), dipentaerythrolpentaacryloyl dihydrogen phosphate (PENTA-P), and ethyl 2-[5-dihydrogen phosphoryl-5.2-dioxa pentyl]acrylate (DHPOBA).

Particularly, these three individual compounds are preferred because of having excellent adhesive property and self-stability of an acrylate compound.

For component (d), at least one bisacrylamide having formula (I) as cited above is comprised.

Such a bisacrylamide provides a superior stability versus hydrolysis (less than 20 % will be normally hydrolyzed, such as in the presence of water and an acid, such as methyl sulfonic acid (MSA) at 50°C in a given period of time) in comparison to the commonly used (meth)acrylates not having at least one phosphorous atom (up to 80 % will be normally hydrolyzed, such as in the presence of water and an acid, such as methyl sulfonic acid (MSA) at 50°C in a given period of time). These commonly used esters of acrylic or methacrylic acid are known in the prior art to be sensible to such environmental conditions for hydrolysis (presence of water and acid). However, hydrolysis stability of (meth)acrylates is problematic in view of the acidity of many dental compositions which severely limits the storage stability of the dental composition. Moreover, hydrolysis taking place under biological conditions in the mouth of the patient is a further concern regarding (meth)acrylates.

Such a bisacrylamide can be exemplarily:

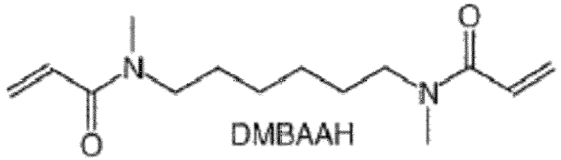

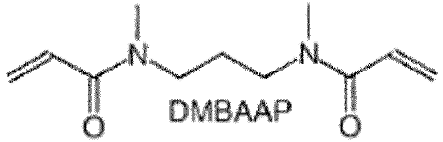

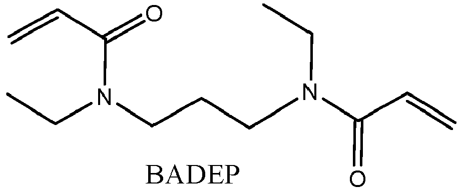

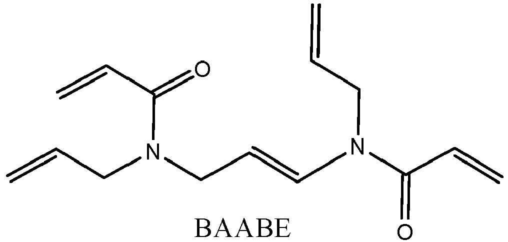

All bisacrylamides according to formula (I) being comprised in the dental RMGI composition (or in all parts of a kit, if a kit is present) are provided in an amount of 10 to 60 weight percent, preferably 15 to 50 weight percent, and more preferably 20 to 40 weight percent based on the total weight of the dental RMGI composition.

In an embodiment, wherein R₂ is a difunctional C₁ to C₁₈ hydrocarbon moiety being substituted with at least one, preferably one or two, acrylamide or acrylate group(s), it is still referred to as "bisacrylamide" in the context of the present invention, even when in case of comprising one or two further acrylamide group(s), it will represent a trisacrylamide or a tetra-acrylamide. In case of comprising at least one acrylate group a "mixed structure" comprising at least two acrylamide groups and at least one ester group is generated.

In one embodiment, the at least one bisacrylamide is selected from the group consisting of N,N'-dimethyl-1,3-bis(acrylamido)-propane, N,N'-dimethyl-1,3-bis(acrylamido)-hexane, N,N'-diethyl-1,3-bis(acrylamido)-propane (BADEP), and N,N'- bisacryloyl-N,N'-bisallyl-1,4-but-2-en-diamine (BAABE).

In one embodiment, the dental resin modified glass-ionomer composition is substantially free, preferably completely free, of ascorbic acid and a salt thereof.

The dental resin modified glass-ionomer composition of the present invention is especially substantially free, preferably completely free, of L(+)ascorbic acid, L(+)-calcium ascorbate, L(+)-sodium ascorbate, dehydroascorbic acid, isoascorbic acid, sodium isoascorbate, (+)-5,6-0-isopropylidene-L-ascorbic acid, 2,6-di-0-palmitoyl-L-ascorbic acid, 6-0-palmitoyl-L-ascorbic acid, D-araboascorbic acid, or the like.

In one embodiment, the dental resin modified glass-ionomer composition further comprises
(e) at least one acrylamide having the following formula (II): 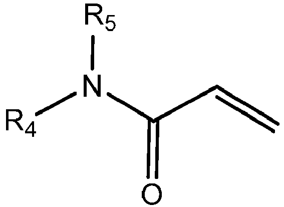 wherein
R₄ = hydrogen, a C₁ to C₁₈ alkyl group, a C₃ to C₁₈ alkylene group comprising at least one carbon-carbon double bond, a C₃ to C₁₈ cycloalkyl group, or a C₅ to C₁₈ aryl group; and
R₅ = hydrogen, a C₁ to C₁₈ alkyl group, a C₃ to C₁₈ alkylene group comprising at least one carbon-carbon double bond, a C₃ to C₁₈ cycloalkyl group, or a C₅ to C₁₈ aryl group.

For component (e), at least one acrylamide having formula (II) as cited above is comprised.

All acrylamides having formula (II) being comprised in the dental RMGI composition (or in all parts of a kit, if a kit is present) are provided in an amount of 2 to 15 weight percent, preferably 2 to 9 weight percent, and more preferably 3 to 5 weight percent based on the total weight of the dental RMGI composition.

A possible example is:

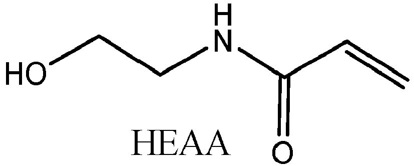

In a preferred embodiment thereof, the at least one acrylamide is selected from the group consisting of N-methylolacrylamide, N-methylolmethacrylamide, N-(2-hydroxyethyl)-methacrylamide, N-methyl-N-(2-hydroxyethyl)-acrylamide, N-methacryloyl-1-aminosalicylic acid, N-acryloyl aspartic acid, and N-methacryloyl glycine.

In one embodiment, the dental resin modified glass-ionomer composition is substantially free, preferably completely free, of a (meth)acrylate not having at least one phosphorous atom.

Such a (meth)acrylate not having at least one phosphorous atom can be exemplarily one of the following known esters:

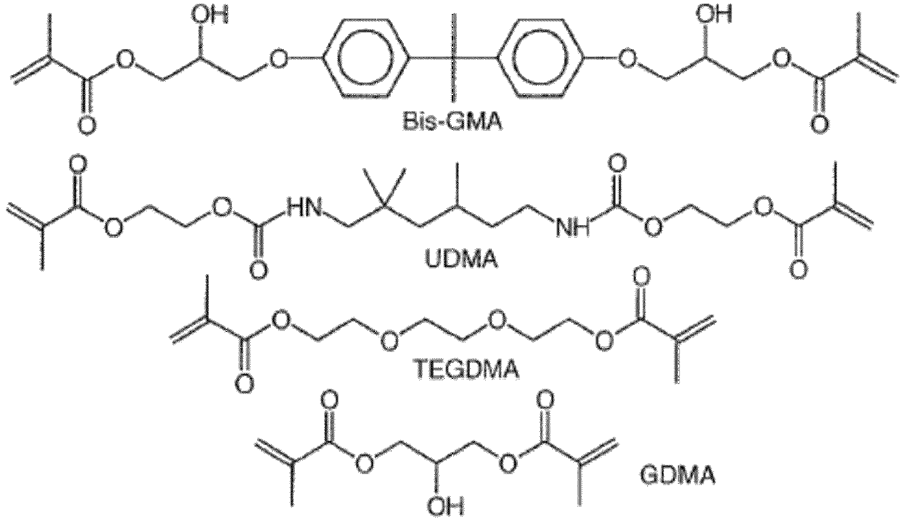

In one embodiment, the dental resin modified glass-ionomer composition further comprises
(f) at least one reactive filler, preferably a fluoroaluminosilicate filler.

Fluoroaluminosilicate fillers are ionically reactive towards acids, such as the polycarboxylic acid (b). As used herein, "ionically reactive" means when the finely divided fluoroaluminosilicate filler (f) and the polycarboxylic acid (b) are mixed together in the presence of water (a), either viscosity increase or hardening of the mixed composition can be observed. According to embodiments of the present invention, the fluoroaluminosilicate filler is a finely divided filler with mean particle size in the range of about 0.02 microns to about 20 microns. The mean particle size of the finely divided fluoroaluminosilicate filler can be measured by a conventional particle size measurement instrument that employs laser light scattering methodology. In one embodiment, the mean particle size of the finely divided fluoroaluminosilicate filler is in the range from about 0.10 microns to about 10 microns. According to one aspect, the finely divided fluoroaluminosilicate filler can further contain calcium, strontium, barium, a rare earth metal, zirconium, zinc, and combinations thereof. Exemplary rare earth metals include, but are not limited to, ytterbium, yttrium, or combinations thereof. In one embodiment, the surface of the fluoroaluminosilicate filler is treated or coated with a coupling agent to enhance the interfacial bonding between the filler and resin matrix and improve mechanical properties. In one embodiment, the coupling agent is a silane compound having at least one polymerizable group selected from the group consisting of an acrylate, a methacrylate, an acrylamido, a methacrylamido, and a vinyl group. Useful examples of coupling agents include, but are not limited to, γ-methacryloyloxypropyl trimethoxysilane (MPTMS), γ-methacryloyloxypropyl triethoxysilane, γ-methacryloyloxypropyl methyldimethoxysilane, vinyltrimethoxysilane, and vinyltriethoxysilane. Another useful example of a coupling agent is a compound that has an acid functional group and a polymerizable group selected from the group consisting of an acrylate, a methacrylate, an acrylamido, a methacrylamido, and a vinyl group. Useful examples include, but are not limited to, acrylic acid, methacrylic acid, and maleic acid.

All fluoroaluminosilicate fillers of component (f) being comprised in the dental RMGI composition (or in all parts of a kit, if a kit is present) are provided in an amount of 10 to 55 weight percent, preferably 15 to 40 weight percent, and more preferably 25 to 35 weight percent based on the total weight of the dental RMGI composition.

In one embodiment, the at least one polycarboxylic acid is a copolymer or homopolymer of at least one of the monomers acrylic acid, methacrylic acid, 2-chloroacrylic acid, 3-chloroacrylic acid, aconitic acid, mesaconic acid, maleic acid, itaconic acid, fumaric acid, glutaconic acid, and citraconic acid.

Such a polycarboxylic acid does not include a polymerizable and ethylenically unsaturated double bond. Furthermore, such a polycarboxylic acid has a weight average molecular weight of 10000 to 130000 g/mol. If the weight average molecular weight is less than 10000 g/mol, the strength of a cured body decreases easily, and the adhesive strength to a tooth tends to decrease. If the weight average molecular weight is more than 130000 g/mol, operativity tends to decrease.

All polycarboxylic acids of component (b) being comprised in the dental RMGI composition (or in all parts of a kit, if a kit is present) are provided in an amount of 2 to 20 weight percent, preferably 2 to 12 weight percent, and more preferably 3 to 7 weight percent based on the total weight of the dental RMGI composition.

In one embodiment, the dental resin modified glass-ionomer composition further comprises
(g) at least one non-reactive filler, preferably a glass flake and/or an inert silica particle.

Examples of fillers that are not ionically reactive towards acidic moieties include, but are not limited to, an inorganic salt, fluoride, glass flake, aluminosilicate glass, aluminoborosilicate glass, quartz, silica, zirconia, zirconia-silica, or a polymeric filler.

Specific examples can be selected from the group consisting of strontium fluoride, ytterbium fluoride, yttrium fluoride, barium sulfate, barium tungstate, zirconium oxide, quartz, inert silica particle, and polymeric filler. Suitable silica particle fillers include fumed silica, colloidal silica, and/or precipitated silica. Examples of silica particle fillers include Aerosil® series such as OX-50, OX-130, and OX-200 silica sold by Degussa (Ridgefield Park, N.J.), and Cab-O-Sil® MS and Cab-O-Sil® TS-530 silica sold by Cabot Corp (Tuscola, III.).

All non-reactive fillers of component (g) being comprised in the dental RMGI composition (or in all parts of a kit, if a kit is present) are provided in an amount of 10 to 50 weight percent, preferably 12 to 35 weight percent, and more preferably 15 to 25 weight percent based on the total weight of the dental RMGI composition.

In one embodiment, the dental resin modified glass-ionomer composition further comprises
(h) at least one polymerization initiator system selected from the group consisting of a photoinitiator system, a redox initiator system, or a combination thereof.

In one embodiment, a photoinitiator is incorporated in the composition. The photoinitiator can be any compound or combination of compounds that can generate free radicals upon exposure to a light source and cause the polymerization or hardening of the composition. The light source can be any dental curing light that emits light in the visible or ultraviolet range. Examples of photoinitiators include, but are not limited to, diketone compounds; benzoin; benzoin ethers and esters; 2,2-diethoxy acetophenone; monoacylphosphine oxide; bisacylphosphine oxide; diaryliodonium salt; triaryl sulfonium salt, silyl glyoxylates; and any mixture thereof. Examples of diketone compounds include, but are not limited to, camphorquinone and 1-phenyl-1,2-propanedione.

Additionally, a co-initiator can be used together with a photoinitiator to enhance curing efficiency. Co-initiators include tertiary amine and sulfinate compounds. Exemplary co-initiators include, but are not limited to, ethyl 4-(N,N-dimethylamino) benzoate; 4-(N,N-dimethylamino) benzoic acid; 4-(N,N-dimethylamino) benzonitrile; 4-(N,N-dimethylamino) benzaldehyde; 2-(ethylhexyl)-4-(N,N-dimethylamino) benzoate; N,N-dimethylaminoethyl methacrylate; N,N-dimethylaminophenethyl alcohol; sodium benzenesulfinate; germanium hydrides (e.g. Ph₃GeH); silicium hydrides, phosphines (e.g. Ph₃P); and sodium toluenesulfinate.

According to one embodiment, the photoinitiator system includes a combination of camphorquinone and a tertiary amine.

All polymerization initiator systems of component (h) being comprised in the dental RMGI composition (or in all parts of a kit, if a kit is present) are provided in an amount of 0.01 to 10 weight percent, preferably 0.2 to 8 weight percent, and more preferably 0.5 to 5 weight percent based on the total weight of the dental RMGI composition.

According to another embodiment, a redox initiator system is incorporated in the dental RMGI composition. A redox initiator system usually comprises at least one reducing agent and at least one oxidizing agent. When the reducing agent and the oxidizing agent are mixed together, a redox reaction proceeds that generates free radicals and initiates the polymerization of monomers, resulting in the curing or hardening of the mixed composition.

The reducing agents include, but are not limited to, aromatic sulfinate salt; aliphatic sulfinate salt; thiourea; substituted thiourea; Fe(II) salt; Cu(I) salt; Co(II) salt; ascorbic acid; ascorbic acid derivatives and salts; barbituric acid; and barbituric acid derivatives and salts.

According to embodiments of the present invention, the reducing agent or agents may be present in an amount from 0.01 to 10 weight percent based on the total weight of the dental RMGI composition.

The oxidizing agents include, but are not limited to, a tertiary hydroperoxide compound with at least one hydroperoxide group attached to at least one tertiary carbon, such as cumene hydroperoxide; a Cu(II) salt, such as Cu(II) acetylacetonate, Cu(II) benzoylacetonate, or Cu(II) cyclohexylbutyrate; a Fe(III) salt, such as FeCl₃, Fe(III) benzoyl acetonate, or Fe(III) cyclohexylbutyrate; a Co(III) salt; persulfate salt; permanganate salt; and combinations of these.

According to embodiments of the present invention, the oxidizing agent or agents may be present in an amount from 0.01 to 10 weight percent based on the total weight of the dental RMGI composition.

The photoinitiator system and the redox initiator system can be incorporated alone or in combination.

Further, the object of the present invention is also solved by a kit comprising such a dental resin modified glass-ionomer composition wherein the kit comprises at least a first part and at least a second part;
wherein the first part comprises
(c) at least one compound having at least one phosphorous atom selected from the group consisting of a (meth)acrylate having at least one phosphate group, a (meth)acrylate having at least one phosphonic acid group, and an acrylic ether having at least one phosphonic acid group; and
(d) at least one bisacrylamide having formula (I);
and wherein the second part comprises
(a) at least water;
(b) at least one polycarboxylic acid; and
(d) at least one bisacrylamide having formula (I).

The provision of a kit comprising such a dental resin modified glass-ionomer composition in a first and a second part maintains at the beginning a separation of various reactive components. Upon mixing of the components of the inventive dental RMGI composition, the resulting mixture is suitable for use in a variety of direct and indirect dental applications, including but not limited to fillings, orthodontic retainers, bridges, space maintainers, tooth replacement appliances, dentures, crowns, posts, jackets, inlays, onlays, facings, veneers, facets, implants, abutments, cements, bonding agents, and splints, and provide improved bonding strengths to dental substrates, such as dentin, enamel, dental alloy, zirconia, ceramic material, or porcelain, and yet maintain other desirable properties.

In a preferred embodiment thereof, the first part further comprises
(f) at least one reactive filler, preferably a fluoroaluminosilicate filler;
and the second part further comprises
(e) at least one acrylamide having formula (II); and
(f) at least one non-reactive filler, preferably a glass flake and/or an inert silica particle.

In a further preferred embodiment thereof, the first and the second part is a paste, respectively; or that the first part is a powder and the second part is a liquid.

The two pastes can be mixed in any volume ratio. In one embodiment, the two pastes are mixed in a volume ratio between about 10: 1 to about 1: 10. In one embodiment, the two pastes are mixed in 1: 1 volume ratio.

The two parts need to be mixed just prior to application, applied to a restoration material, and hardened inside a patient's mouth by self-curing or the combination of self-curing or light-curing.

Commonly, it is done manually by a spatula or by applying a mounted static mixer as mixing tip arranged at the exit of a double cartridge, which automatically blends the two parts of the kit prior to application.

The present invention thus addresses the problem of providing an enhanced adhesive strength towards tooth structure, and a simplified application procedure.

The following non-limiting examples are provided to illustrate an embodiment of the present invention and to facilitate understanding of the invention but are not intended to limit the scope of the invention, which is defined by the claims appended hereto.

All inventive and comparative experiments have been executed by making use of dental resin modified glass-ionomer compositions wherein the first and the second part is a paste.

All values given in Tables 1, 2, 5, and 6 are given in weight percentages of the respective oxidizing paste or reducing paste.

All components of the respective oxidizing paste or reducing paste together provide a total sum of 100 weight percent for each individual paste.

### Preparation:

For preparation of the reducing paste. PAA was first solved in distilled water by pre-mixing in the SpeedMixer DAC 600-2 VAC-P (Hauschild & Co. KG. E-QC-1936). All other components according to the tables were directly weighed into a 50 mL plastic container per paste (PP 30. Hauschild & Co. KG). For preparation of oxidizing paste also all components according to the tables were directly weighed into a 50 mL plastic container per paste (PP 30. Hauschild & Co. KG). Solid components were put in first. liquids last; batch sizes per paste was 15-45 g. Each container was subsequently closed with a lid with a hole in it and placed in the SpeedMixer. Mixing was conducted twice at 2500 rpm for 2 min and once at 1000 rpm/100 mbar for 1 min. In between the mixing steps. Pastes were briefly stirred with a spatula. Finally. the hole in the lid was closed with a scotch tape and containers are stored at room temperature until further use.

For evaluation both pastes were manually filled into double-barrel syringes allowing a 1:1 volume ratio and placed in the SpeedMixer again. Mixing was conducted three times at 1000 rpm for 1 min to remove residual air-bubbles. Long mixing tips (MixPac) as used e.g. for RelyX Luting Plus were utilized to achieve homogenous extrusion of the luting cement.

### Shear bond strength (SBS):

To determine the bond strength of the respective luting cement on dentin/enamel. Extracted human molars were embedded into a cylindrical form in a cold-cured matrix resin and finally wet ground to expose flat surfaces using 320 and 600 grit abrasive paper from buccal/lingual side. Stainless steel rods (2.985 mm in diameter) were sandblasted. Ultrasonically cleaned and dried. The respective cement was applied to the surface of the steel rod and placed onto dentin/enamel and allowed to self-cure at 37 °C/50% R.H. for 5 minutes under 220g load. The specimens (n = 6 per group) were stored in 37 °C water for 24-hr. Shear bond strength (SBS) was obtained with a Zwick at crosshead speed of 1 mm/min.

### Working time (WT):

A bead-like body was formed which was periodically probed with a metal instrument at 23°C. The end of working time was defined by the transition point from viscous. spreadable material to elastic. gel-like one; the start of working time by the beginning of hand-mixing.

### Setting time (ST):

The setting time of each material was measured according to ISO 9917-2.

### 3-point bending:

Mechanical data of flexural strength (FS) and flexural modulus (E-Mod.) was measured in 3-point bending mode according to ISO 9917-2.

### Opacity:

The opacity of each material was investigated using following procedure (n = 2): Place a piece of film on one of the metal plates and place the mould upon it. Slightly overfill the mould with the respective material prepared in accordance with the manufacturer's instruction. Place a second piece of film on the material in the mould and cover this with the second metal plate. Thus displacing excess material. Clamp the mould together and transfer the assembly immediately to the oven maintained at 37 ± 2 °C and ≥ 95% R.H. After 60 min. timed from the start of the mixing, remove the specimen from the mould and finish the periphery of the specimen to remove flash and irregularities. Afterwards, place the specimen in distilled water in the dark at 60 °C for 24 hours. Finally. the opacity of each specimen was measured using a Datacolor 800.

### Inventive Examples

**Table 1: Components of the respective first part (oxidizing paste) of the dental resin modified glass-ionomer compositions of inventive examples 1 to 5.**

| **Oxidizing Paste** | **Example 1** | **Example 2** | **Example 3** | **Example 4** | **Example 5** |
|---|---|---|---|---|---|
| BADEP | 16.46 | 12.82 | 12.83 | - | - |
| BAABE | - | - | - | 16.47 | 16.47 |
| MDP | 15.50 | 18.75 | 9.37 | 15.50 | - |
| PENTA | - | - | 9.37 | - | - |
| DHPOBA | - | - | - | - | 15.50 |
| FAS Filler | 48.00 | 56.00 | 56.00 | 56.00 | 56.00 |
| Strontium Fluoride | 18.00 | - | - | - | - |
| Ytterbium Floride | - | 10.00 | 10.00 | 10.00 | 10.00 |
| Cumene hydroperoxide | 2.00 | 2.40 | 2.40 | 2.00 | 2.00 |
| 2-Methoxy-4-methylphenol | 0.03 | 0.03 | 0.03 | 0.03 | 0.03 |

**Table 2: Components of the respective second part (reducing paste) of the dental resin modified glass-ionomer compositions of inventive examples 1 to 5.**

| **Reducing Paste** | **Example 1** | **Example 2** | **Example 3** | **Example 4** | **Example 5** |
|---|---|---|---|---|---|
| Polyacrylic acid | 11.15 | 9.60 | 9.60 | 11.15 | 11.15 |
| Distilled water | 16.80 | 15.30 | 15.30 | 16.80 | 16.80 |
| BADEP | 50.59 | 38.80 | 38.80 | - | - |
| BAABE | - | - | - | 50.60 | 50.60 |
| HEAA | - | 8.25 | 8.25 | - | - |
| Benzoylthiourea | 2.00 | 1.00 | 1.00 | 2.00 | 2.00 |
| Glass flakes | - | 27.00 | 27.00 | - | - |
| Inert silica particle | 19.45 | - | - | 19.45 | 19.45 |

**Table 3: Experimental data of the dental resin modified glass-ionomer compositions of inventive examples 1 to 5.**

| | **Example 1** | **Example 2** | **Example 3** | **Example 4** | **Example 5** |
|---|---|---|---|---|---|
| Working Time [s] | 205 | 163 | 110 | 135 | 50 |
| Setting Time [s] | 150 | 135 | 105 | 110 | 70 |
| Flexural Strength [MPa] | 25.0 | 31.7 | 35.4 | 28.6 | 34.5 |
| Flexural Modulus [MPa] | 1591 | 1897 | 2402 | 1241 | 1733 |
| Opacity [%] | 35 | 19 | 19 | 39 | 42 |

**Table 4: Experimental results of the dental resin modified glass-ionomer compositions of inventive examples 1 to 5.**

| | **Example 1** | **Example 2** | **Example 3** | **Example 4** | **Example 5** |
|---|---|---|---|---|---|
| SBS [MPa], Dentin | 11.5 | 12.1 | 14.2 | 12.1 | 16.3 |
| SBS [MPa], Enamel | 19.2 | 24.2 | 20.2 | 20.0 | 21.8 |

### Comparative Examples

**Table 5: Components of the respective first part (oxidizing paste) of the dental resin modified glass-ionomer compositions of comparative examples 6 to 10.**

| **Oxidizing Paste** | **Example 6** | **Example 7** | **Example 8** | **Example 9** | **Example 10** |
|---|---|---|---|---|---|
| BADEP | - | - | 12.82 | - | - |
| BAABE | - | - | - | 16.47 | - |
| TEGDMA | 16.47 | 16.47 | - | - | 16.47 |
| HEAA | - | - | 18.75 | 15.50 | 15.50 |
| MDP | 15.50 | - | - | - | - |
| DHPOBA | - | 15.50 | - | - | - |
| FAS Filler | 56.00 | 56.00 | 56.00 | 56.00 | 56.00 |
| Ytterbium Floride | 10.00 | 10.00 | 10.00 | 10.00 | 10.00 |
| Cumene hydroperoxide | 2.00 | 2.00 | 2.40 | 2.00 | 2.00 |
| 2-Methoxy-4-methyl phenol | 0.03 | 0.03 | 0.03 | 0.03 | 0.03 |

**Table 6: Components of the respective second part (reducing paste) of the dental resin modified glass-ionomer compositions of comparative examples 6 to 10.**

| **Reducing Paste** | **Example 6** | **Example 7** | **Example 8** | **Example 9** | **Example 10** |
|---|---|---|---|---|---|
| Polyacrylic acid | 11.15 | 11.15 | 9.60 | 11.15 | 11.15 |
| Distilled water | 16.80 | 16.80 | 15.30 | 16.80 | 16.80 |
| BADEP | - | - | 38.85 | - | - |
| BAABE | - | - | - | 50.60 | - |
| TEGDMA | 50.60 | 50.60 | - | - | 50.60 |
| HEAA | - | - | 8.25 | - | - |
| Benzoylthiourea | 2.00 | 2.00 | 1.00 | 2.00 | 2.00 |
| Glass flakes | - | - | 27.00 | - | - |
| Inert silica particle | 19.45 | 19.45 | - | 19.45 | 19.45 |

**Table 7: Experimental data of the dental resin modified glass-ionomer compositions of comparative examples 6 to 10.**

| | **Example 6** | **Example 7** | **Example 8** | **Example 9** | **Example 10** |
|---|---|---|---|---|---|
| Working Time [s] | 50 | 30 | 313 | 255 | 210 |
| Setting Time [s] | 60 | 75 | 125 | 145 | 130 |
| Flexural Strength [MPa] | 36.2 | 33.1 | 31.4 | 23.4 | 11.5 |
| Flexural Modulus [MPa] | 1555 | 2101 | 2151 | 1352 | 679 |
| Opacity [%] | 34 | 41 | 20 | 43 | 52 |

**Table 8: Experimental results of the dental resin modified glass-ionomer compositions of comparative examples 6 to 10.**

| | **Example 6** | **Example 7** | **Example 8** | **Example 9** | **Example 10** |
|---|---|---|---|---|---|
| SBS [MPa], Dentin | 6.0 | 2.30 | 0.82 | 0.90 | 0.10 |
| SBS [MPa], Enamel | 12.4 | 11.7 | 0.46 | 1.20 | 0.30 |

While the principles of the invention have been explained in relation to certain particular embodiments, and are provided for purposes of illustration, it is to be understood that various modifications thereof will become apparent to those skilled in the art upon reading the specification. Therefore, it is to be understood that the invention disclosed herein is intended to cover such modifications as fall within the scope of the appended claims. The scope of the invention is limited only by the scope of the appended claims.

## Claims

1. Dental resin modified glass-ionomer composition **characterized in that** the dental resin modified glass-ionomer composition comprises
(a) water;
(b) at least one polycarboxylic acid;
(c) at least one compound having at least one phosphorous atom selected from the group consisting of a (meth)acrylate having at least one phosphate group, a (meth)acrylate having at least one phosphonate group, a (meth)acrylamide having at least one phosphate group, a (meth)acrylamide having at least one phosphonate group, an acrylic ether having at least one phosphate group, and an acrylic ether having at least one phosphonate group; and
(d) at least one bisacrylamide having the following formula (I): 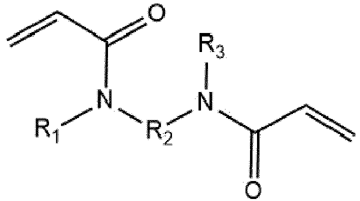 wherein
R₁ = hydrogen, a C₁ to C₁₈ alkyl group, a C₃ to C₁₈ alkylene group comprising at least one carbon-carbon double bond, a C₃ to C₁₈ cycloalkyl group, or a C₅ to C₁₈ aryl group;
R₂ = a difunctional C₁ to C₁₈ alkyl group, a difunctional C₄ to C₁₈ alkylene group comprising at least one carbon-carbon double bond, a difunctional C₃ to C₁₈ cycloalkyl group, a difunctional C₅ to C₁₈ aryl group, or a difunctional C₁ to C₁₈ hydrocarbon moiety being substituted with at least one, preferably one or two, acrylamide or acrylate group(s); and
R₃ = hydrogen, a C₁ to C₁₈ alkyl group, a C₃ to C₁₈ alkylene group comprising at least one carbon-carbon double bond, a C₃ to C₁₈ cycloalkyl group, or a C₅ to C₁₈ aryl group.

2. Dental resin modified glass-ionomer composition according to claim 1 **characterized in that** the at least one compound having at least one phosphorous atom is selected from the group consisting of 2-(meth)acryloyloxyethyldihydrogen phosphate, bis[2-(meth)acryloyloxyethyl]hydrogen phosphate, 2-(meth)acryloyloxyethylphenylhydrogen phosphate, 6-(meth)acryloyloxyhexyldihydrogen phosphate, 6-(meth)acryloyloxyhexylphenylhydrogen phosphate, 10-(meth)acryloyloxydecyldihydrogen phosphate, 1,3-di(meth)acryloylpropane-2-dihydrogen phosphate, 1,3-di(meth) acryloylpropane-2-phenylhydrogen phosphate, dipentaerythrolpentaacryloyl dihydrogen phosphate, ethyl 2-[5-dihydrogen phosphoryl-5.2-dioxa pentyl]acrylate, and bis[5-{2-(meth)acryloyloxyethoxycarbonyl} heptyl]hydrogen phosphate.

3. Dental resin modified glass-ionomer composition according to claim 2 **characterized in that** at least one compound having at least one phosphorous atom is selected from the group consisting of 10-methacryloyloxydecyldihydrogen phosphate, dipentaerythrolpentaacryloyl dihydrogen phosphate, and ethyl 2-[5-dihydrogen phosphoryl-5.2-dioxa pentyl]acrylate.

4. Dental resin modified glass-ionomer composition according to one of the preceding claims **characterized in that** the dental resin modified glass-ionomer composition is substantially free, preferably completely free, of ascorbic acid and a salt thereof.

5. Dental resin modified glass-ionomer composition according to one of the preceding claims **characterized in that** the dental resin modified glass-ionomer composition is substantially free, preferably completely free, of a (meth)acrylate not having at least one phosphorous atom.

6. Dental resin modified glass-ionomer composition according to one of the preceding claims **characterized in that** the dental resin modified glass-ionomer composition further comprises
(e) at least one acrylamide having the following formula (II): 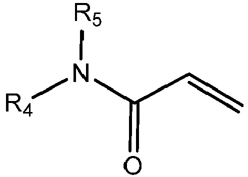 wherein
R₄ = hydrogen, a C₁ to C₁₈ alkyl group, a C₃ to C₁₈ alkylene group comprising at least one carbon-carbon double bond, a C₃ to C₁₈ cycloalkyl group, or a C₅ to C₁₈ aryl group; and
R₅ = hydrogen, a C₁ to C₁₈ alkyl group, a C₃ to C₁₈ alkylene group comprising at least one carbon-carbon double bond, a C₃ to C₁₈ cycloalkyl group, or a C₅ to C₁₈ aryl group.

7. Dental resin modified glass-ionomer composition according to claim 6 **characterized in that** at least one acrylamide is selected from the group consisting of N-methylolacrylamide, N-methylolmethacrylamide, N-(2-hydroxyethyl)-methacrylamide, N-methyl-N-(2-hydroxyethyl)-acrylamide, N-methacryloyl-1-aminosalicylic acid, N-acryloyl aspartic acid, and N-methacryloyl glycine.

8. Dental resin modified glass-ionomer composition according to one of the preceding claims **characterized in that** at least one bisacrylamide is selected from the group consisting of N,N'-dimethyl-1,3-bis(acrylamido)-propane, N,N'-dimethyl-1,3-bis(acrylamido)-hexane, N,N'-diethyl-1,3-bis(acrylamido)-propane, and N,N'-bisacryloyl-N,N'-bisallyl-1,4-but-2-endiamine.

9. Dental resin modified glass-ionomer composition according to one of the preceding claims **characterized in that** the dental resin modified glass-ionomer composition further comprises
(f) at least one reactive filler, preferably a fluoroaluminosilicate filler.

10. Dental resin modified glass-ionomer composition according to one of the preceding claims **characterized in that** the at least one polycarboxylic acid is a copolymer or homopolymer of at least one of the monomers acrylic acid, methacrylic acid, 2-chloroacrylic acid, 3-chloroacrylic acid, aconitic acid, mesaconic acid, maleic acid, itaconic acid, fumaric acid, glutaconic acid, and citraconic acid.

11. Dental resin modified glass-ionomer composition according to one of the preceding claims **characterized in that** the dental resin modified glass-ionomer composition further comprises
(g) at least one non-reactive filler, preferably a glass flake and/or an inert silica particle.

12. Dental resin modified glass-ionomer composition according to one of the preceding claims **characterized in that** the dental resin modified glass-ionomer composition further comprises
(h) at least one polymerization initiator system selected from the group consisting of a photoinitiator system, a redox initiator system, or a combination thereof.

13. Kit comprising a dental resin modified glass-ionomer composition according to one of the preceding claims **characterized in that** the kit comprises at least a first part and at least a second part;
wherein the first part comprises
(c) at least one compound having at least one phosphorous atom selected from the group consisting of a (meth)acrylate having at least one phosphate group, a (meth)acrylate having at least one phosphonic acid group, and an acrylic ether having at least one phosphonic acid group; and
(d) at least one bisacrylamide having formula (I) of claim 1;
and wherein the second part comprises
(a) at least water;
(b) at least one polycarboxylic acid; and
(d) at least one bisacrylamide having formula (I) of claim 1.

14. Kit according to claim 13 **characterized in that**
the first part further comprises
(f) at least one reactive filler, preferably a fluoroaluminosilicate filler;
and the second part further comprises
(e) at least one acrylamide having formula (II) of claim 6; and
(f) at least one non-reactive filler, preferably a glass flake and/or an inert silica particle.

15. Kit according to claim 13 or 14 **characterized in that** the first and the second part is a paste, respectively; or that the first part is a powder and the second part is a liquid.
